# EUROPÄISCHE PATENTSCHRIFT

(11) **EP 0 568 496 B1**
(45) Veröffentlichungstag und Bekanntmachung des Hinweises auf die Patenterteilung: **30.12.1998**
(21) Anmeldenummer: 93810284.5
(22) Anmeldetag: 20.04.1993
(51) Int. Cl.: G03F 7/039, G03F 7/023, C08F 12/22, C07C 43/215, C07C 43/225

(54) **Photoresistmaterial auf Basis von Polystyrolen**
Photoresist materials based on polystyrene
Matériaux photorésists à base de polystyrènes

(30) Priorität: 29.04.1992 CH 1378/92
(43) Veröffentlichungstag der Anmeldung: 03.11.1993
(73) Patentinhaber: Olin Microelectronic Chemicals, Inc., Norwalk, Connecticut 06856-4500 (US)
(72) Erfinder: Steinmann, Alfred, Dr., CH-1724 Praroman (CH)
(74) Vertreter: Klunker . Schmitt-Nilson . Hirsch

(56) Entgegenhaltungen:
- EP-A- 0 355 652
- US-A- 5 084 490

## Beschreibung

Die vorliegende Erfindung betrifft neue Styrolderivate, die daraus hergestellten Homo- und Copolymeren, strahlungsempfindliche Zusammensetzungen enthaltend diese Polymeren sowie die Verwendung solcher Zusammensetzungen als Photoresists zur Herstellung von gedruckten Schaltkreisen.

Positiv- und Negativphotoresistzusammensetzungen auf Basis von Poly[p-tert.-butoxycarbonyloxystyrol] und Onium-Salzen, die für die Lithographie im tiefen UV-Bereich (DUV, Wellenlängen von ca. 200-300 nm) geeignet sind, sind beispielsweise aus dem US-Patent 4,491,628 bekannt.

Ähnliche Resistformulierungen enthaltend Poly[p-2-tetrahydropyranyloxy]styrol werden in der EP-A 342498 beschrieben.

Diese Zusammensetzungen erfordern jedoch relativ hohe Belichtungsenergien, was insbesondere bei der industriellen Herstellung von gedruckten Schaltungen von Nachteil ist.

In dem Aufsatz "Tetrahydropyranyl- and Furanyl-Protected Polyhydroxystyrene in Chemical Amplification Systems" von S.A.M. Hesp, N. Hayashi und T. Ueno in J. Appl. Pol. Sci. 42, 877 (1991) wird offenbart, dass sich Poly[p-(tetrahydrofuranyloxy)styrol] und Poly[p-(tetrahydropyranyloxy)styrol] zur Herstellung von Positiv-Photoresists mit hoher Sensitivität und Auflösungen im Submikronbereich eignen. Die dort beschriebenen Photoresists können jedoch nicht rein wässrig-alkalisch, sondern nur unter Zusatz von n-Propanol, entwickelt werden.

Es wurde nun gefunden, dass wässrig-alkalisch entwickelbare Positiv-Photoresists mit hoher Sensitivität erhalten werden, wenn man Polystyrole als Basisharze verwendet, deren aromatische Kerne durch zwei oder drei säurespaltbare Gruppen substituiert sind.

Gegenstand des vorliegenden Erfindung sind Verbindungen der Formel (I) gemäß Anspruch 1, Polymere gemäß Anspruch 4, im Verfahren zum Herstellen dieser Polymere gemäß Anspruch 8, eine Strahlungsempfindliche Zusammensetzung gemäß Anspruch 9 und ihre Verwendung gemäß Anspruch 12.

Gegenstand der vorliegenden Erfindung sind Verbindungen der Formel(I) 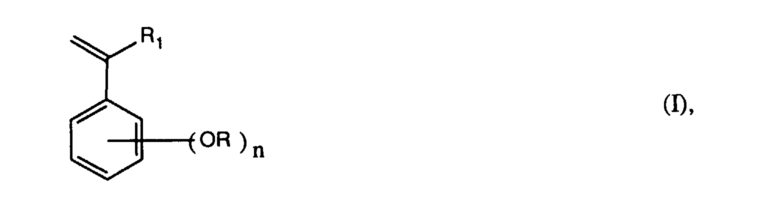 worin R₁ für Wasserstoff, Methyl oder Halogen steht, n 2 oder 3 bedeutet und R für C₁-C₆-Alkyl, Benzyl, 2-Tetrahydrofuranyl, 2-Tetrahydropyranyl, C₁-C₆-Alkoxycarbonyl, Phenoxycarbonyl oder Benzyloxycarbonyl steht, oder, falls zwei der Substituenten OR in ortho-Stellung zueinander stehen, zwei Reste R zusammen eine Ethylengruppe, die gegebenenfalls durch bis zu vier C₁-C₆-Alkylgruppen substituiert ist, oder eine C₂-C₆-Alkylidengruppe bilden.

R₁ als Halogen ist vorzugsweise Chlor oder Brom.

Steht R für C₁-C₆-Alkyl oder C₁-C₆-Alkoxycarbonyl, so handelt es sich um geradkettige oder bevorzugt um verzweigte Reste.

Beispiele für C₁-C₆-Alkylreste sind Methyl, Ethyl, n-Propyl, i-Propyl, n-Butyl, n-Pentyl, neo-Pentyl, n-Hexyl oder vorzugsweise t-Butyl.

Beispiele für C₁-C₆-Alkoxycarbonylgruppen sind Methoxycarbonyl, Ethoxycarbonyl, n-Propoxycarbonyl, i-Propoxycarbonyl, n-Butoxycarbonyl, n-Pentoxycarbonyl, neo-Pentoxycarbonyl, n-Hexoxycarbonyl oder insbesondere t-Butoxycarbonyl.

Gegebenenfalls substituierte Ethylengruppen sind beispielsweise Ethylen, Propylen, 1,2-Dimethylethylen und Tetramethylethylen.

Beispiele für C₂-C₆-Alkylidengruppen sind Ethyliden, Propyliden und Isopropyliden.

Bevorzugt sind Verbindungen der Formel (I), worin R₁ Wasserstoff ist, n 2 bedeutet und die beiden Substituenten OR in 3,4- oder 3,5-Stellung zur Vinylgruppe stehen.

Besonders bevorzugt sind Verbindungen der Formel (I), worin R₁ Wasserstoff ist, n 2 bedeutet, die beiden Substituenten OR in 3,4- oder 3,5-Stellung zur Vinylgruppe stehen und R für tert.-Butoxycarbonyl, 2-Tetrahydrofuranyl oder 2-Tetrahydropyranyl steht.

Die Verbindungen der Formel (I) können beispielsweise nach bekannten Methoden aus den Di- oder Trihydroxyverbindungen der Formel (II) 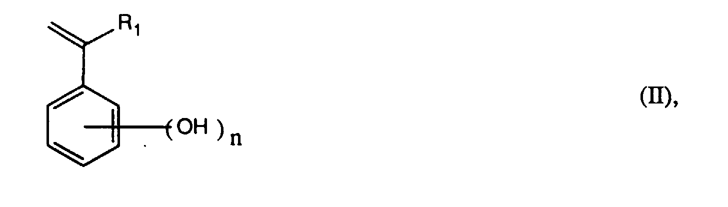 worin R₁ und n die gleiche Bedeutung wie in Formel (I) haben, synthetisiert werden.

Die Verbindungen der Formel (I), worin R für C₁-C₆-Alkyl oder Benzyl steht, können z.B. durch Umsetzung von Verbindungen der Formel (II) mit den entsprechenden Alkyl- oder Benzylhalogeniden oder -tosylaten oder Dialkyl- bzw. Dibenzylsulfaten in alkalischer Lösung erhalten werden.

Die Verbindungen der Formel (I), worin R für C₁-C₆-Alkoxycarbonyl, Phenoxycarbonyl oder Benzyloxycarbonyl steht, sind aus den Verbindungen der Formel (II) durch Umsetzung mit den entsprechenden Dialkyl-, Diphenyl- bzw. Dibenzylcarbonaten in Gegenwart von Basen erhältlich.

Die Verbindungen der Formel (I), worin R 2-Tetrahydrofuranyl oder 2-Tetrahydropyranyl bedeutet, können aus den Verbindungen der Formel (II) durch Reaktion mit 2,3-Dihydrofuran bzw. 2,3-Dihydropyran in saurer Lösung hergestellt werden.

Die Verbindungen der Formel (I), worin zwei Reste R zusammen eine Ethylengruppe, die gegebenenfalls durch bis zu vier C₁-C₆-Alkylgruppen substituiert ist, oder eine C₂-C₆-Alkylidengruppe bilden, sind aus den Verbindungen der Formel (II) mit zwei ortho-ständigen OH-Gruppen durch Umsetzung mit den entsprechenden Aldehyden oder Ketonen unter sauren Bedingungen zugänglich.

Die Verbindungen der Formel (II) können beispielsweise in einer mehrstufigen Synthese aus den Di- bzw. Trihydroxybenzoesäuren der Formel (III) 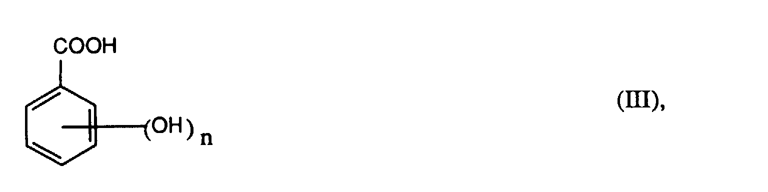 worin n 2 oder 3 bedeutet, gewonnen werden.
Dabei werden im ersten Schritt die Di- bzw. Trihydroxybenzoesäuren der Formel (III) mit Acetanhydrid zu den entsprechenden Di- bzw. Triacetoxybenzoesäuren umgesetzt und anschliessend mit Thionylchlorid in die Säurechloride der Formel (IV) überführt 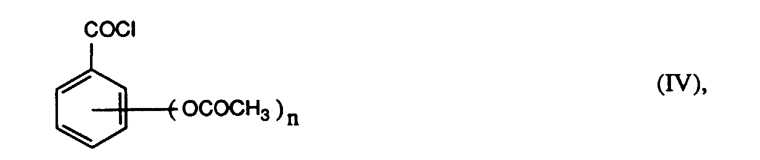 worin n 2 oder 3 bedeutet.
Aus den Säurechloriden der Formel (IV) können durch Reaktion mit tertiären Aminen und Alkenen der Formel (V) 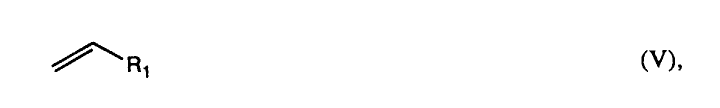 worin R₁ die gleiche Bedeutung wie in Formel (I) hat, die Di- bzw. Triacetoxystyrole der Formel (VI) synthetisiert werden 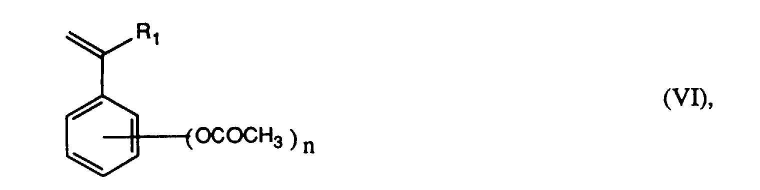 worin R₁ und n die gleiche Bedeutung wie in Formel (I) haben, welche schliesslich in bekannter Weise zu den Di- bzw. Trihydroxystyrolen der Formel (II) verseift werden können.

In einigen Fällen lassen sich die Verbindungen der Formel (II) auch durch weniger aufwendige Synthesen herstellen. So kann z.B 3,4-Dihydroxystyrol durch Decarboxylierung der kommerziell erhältlichen Kaffeesäure (3,4-Dihydroxyzimtsäure) synthetisiert werden.

Die erfindungsgemässen Verbindungen der Formel (I) können, gegebenenfalls in Gegenwart von olefinisch ungesättigten Comonomeren, in bekannter Weise polymerisiert werden.

Gegenstand vorliegender Erfindung sind somit Polymere mit einem Molekulargewicht M_{w} von 10³ bis 10⁶ (gemessen mittels Gelpermeationschromatographie) enthaltend, bezogen auf die Gesamtmenge der im Polymer vorhandenen Strukturelemente, 100 bis 5 mol% eines wiederkehrenden Strukturelementes der Formel (VII) 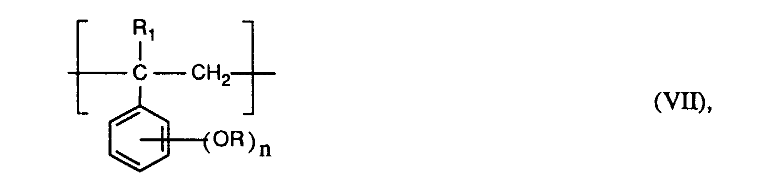 worin R₁, R und n die oben angegebene Bedeutung haben,
und 95 bis 0 mol% eines wiederkehrenden Strukturelementes der Formel (VIII) 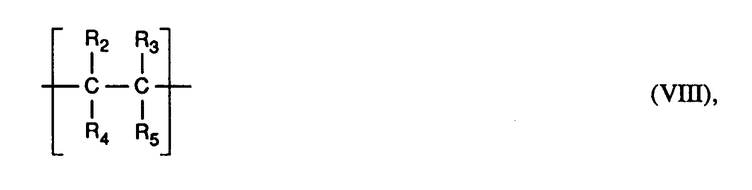 worin R₂ und R₃ unabhängig voneinander jeweils Wasserstoff, unsubstituiertes oder durch Halogenatome, Cyano- oder Nitrogruppen substituiertes C₁-C₄-Alkyl oder ein unsubstituiertes oder durch Halogenatome, C₁-C₄-Alkoxy-, Hydroxy-, Cyano- oder Nitrogruppen substituiertes Phenyl oder Naphthyl bedeuten, und R₄ und R₅ unabhängig voneinander je ein Wasserstoff- oder Halogenatom, unsubstituiertes oder durch Halogenatome, Cyano- oder Nitrogruppen substituiertes C₁-C₁₂-Alkyl, unsubstituiertes oder durch Halogenatome, Hydroxy-, Cyano-, Nitrogruppen, C₁-C₄-Alkyl oder C₁-C₄-Alkoxy substituiertes Phenyl, Naphthyl oder Benzyl oder einen der folgenden Reste -OR₆, -COOR7, -COR₈ oder zusammen einen zweiwertigen Rest der Formeln (IX)-(XI) bedeuten, 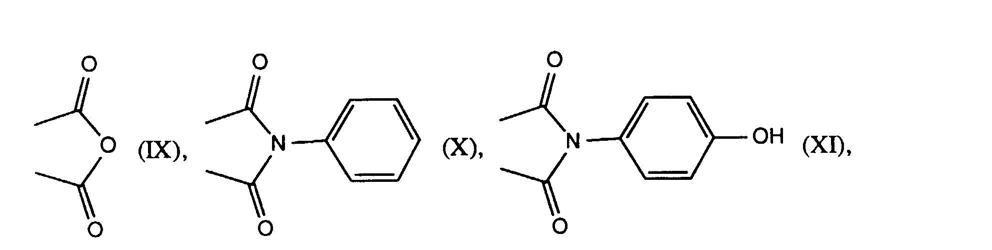 wobei R₆ und R₇ unabhängig voneinander je ein Wasserstoffatom, unsubstituiertes oder durch Halogenatome, Cyano- oder Nitrogruppen substituiertes C₁-C₁₂-Alkyl, unsubstituiertes oder durch Halogen, Cyano-, Nitrogruppen, C₁-C₄-Alkyl oder C₁-C₄-Alkoxy substituiertes Phenyl oder Naphthyl bedeuten und R₈ die gleiche Bedeutung wie R₆ hat oder für den Rest 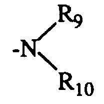 steht, worin R₉ und R₁₀ unabhängig voneinander die gleiche Bedeutung wie R₈ haben.

Bedeuten R₂, R₃, R₄ und R₅ im Strukturelement der Formel (VIII) ein Alkyl, so handelt es sich dabei um geradkettige oder verzweigte, bevorzugt um geradkettige Alkylreste.

Halogenatome als Substituenten sind bevorzugt Chlor oder Brom.

Als gegebenenfalls substituiertes Alkyl kommen beispielsweise Methyl, Ethyl, 2-Chlorethyl, 2-Nitroethyl, n-Propyl, Isopropyl, n-Butyl, sek.-Butyl, tert.-Butyl, n-Pentyl, Isoamyl, n-Hexyl, 2-Ethylhexyl, n-Decyl, 6-Nitrohexyl oder 9-Bromnonyl in Frage.

Beispiele für substituiertes Phenyl oder Naphthyl sind o-, m- oder p-Chlorphenyl, o-, m-oder p-Tolyl, Xylyl, 2-Nitrophenyl oder α-Chlornaphthyl.

Im Strukturelement der Formel (VIII) bedeuten R₂, R₃ und R₄ unabhängig voneinander vorzugsweise ein Wasserstoffatom, C₁-C₆-Alkyl oder Phenyl und bedeutet R₅ vorzugsweise ein Halogenatom, Phenyl oder Benzyl oder einen der Reste -OR₆, -COOR₇ oder -COR₈, worin R₆, R₇ und R₈ unabhängig voneinander je ein Wasserstoffatom, C₁-C₆-Alkyl oder Phenyl bedeuten und R₈ ausserdem für den Rest 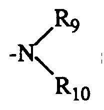 stehen kann, worin R₉ und R₁₀ unabhängig voneinander die bevorzugte Bedeutung von R₆ haben.

Die erfindungsgemässen Polymeren weisen vorzugsweise ein Molekulargewicht M_{w} von 5'000 bis 500'000, insbesondere von 20'000 bis 150'000, auf.

Ferner enthalten die erfindungsgemässen Polymeren vorzugsweise 100 bis 20 mol%, insbesondere 100 bis 50 mol%, eines wiederkehrenden Strukturelementes der Formel (VII) und 80 bis 0 mol%, insbesondere 50 bis 0 mol%, eines wiederkehrenden Strukturelementes der Formel (VIII).

Besonders bevorzugt sind Polymere enthaltend 100 mol% eines wiederkehrenden Strukturelements der Formel (VII).

Insbesondere bevorzugt sind erfindungsgemässe Polymere, worin in der Formel (VII) R₁ Wasserstoff ist, n 2 bedeutet und die beiden Substituenten OR in 3,4- oder 3,5-Stellung zur Vinylgruppe stehen.

Der Rest R in der Formel (VII) steht vorzugsweise für tert.-Butoxycarbonyl, 2-Tetrahydrofuranyl oder 2-Tetrahydropyranyl.

Einen weiteren Erfindungsgegenstand bildet ein Verfahren zur Herstellung von Polymeren enthaltend, bezogen auf die Gesamtmenge der im Polymer vorhandenen Strukturelemente, 100 bis 5 mol% eines wiederkehrenden Strukturelementes der Formel (VII) und 95 bis 0 mol% eines wiederkehrenden Strukturelementes der Formel (VIII), dadurch gekennzeichnet, dass man eine Verbindung der Formel (I) oder ein Gemisch aus einer Verbindung der Formel (I) und darin bis zu 95 mol% enthaltenen Verbindungen der Formel (XII) 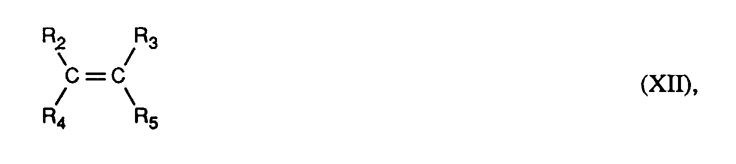 worin R₂, R₃, R₄ und R₅ die oben angegebene Bedeutung haben, radikalisch polymerisiert.

Die radikalische Polymerisation kann unter Anwendung verschiedener Techniken durchgeführt werden. Diese sind beispielsweise von S. Sandler und W. Karo in "Polymer Synthesis" Vol. 1, S. 3-17, 1968, Academic Press, New York, beschrieben worden. Übliche Polymerisationsverfahren sind beispielsweise Polymerisation in der Masse oder in Lösungsmitteln, Emulsions-, Suspensions- oder Fällungspolymerisation.

Die Verbindungen der Formel (XII) sind bekannt und zum Teil im Handel erhältlich. Neben Olefinen, wie beispielsweise Ethylen oder Propylen, sind als Beispiele für Verbindungen der Formel (XII) insbesondere die Vinylverbindungen zu nennen. Beispiele solcher Monomere sind die Styroltypen, wie beispielsweise Styrol, α-Methylstyrol, p-Methylstyrol, p-Hydroxystyrol, p-Acetylstyrol oder p-Hydroxyphenylstyrol, α,β-ungesättigte Säuren, deren Ester oder Amide, wie beispielsweise Acrylsäure, Acrylsäuremethylester, Acrylsäureamid, die entsprechenden Methacrylverbindungen, Maleinsäure, Maleinsäuremethylester, Maleinimid, p-Hydroxyphenylmaleinimide oder 4-Vinylbenzoesäure-tert.-butylester, halogenhaltige Vinylverbindungen, wie beispielsweise Vinylchlorid, Vinylfluorid, Vinylidenchlorid oder Vinylidenfluorid, Vinylester, wie beispielsweise Vinylacetat oder Vinylether, wie beispielsweise Methylvinylether oder Butylvinylether.

Weitere geeignete Verbindungen sind beispielsweise die Allylverbindungen, wie Allylchlorid, Allylbromid oder Allylcyanid.

Die Polymerisation wird in der Regel durch einen üblichen Radikalstarter initiert. Dazu zählen thermische Initiatoren, wie Azoverbindungen, beispielsweise Azoisobutyronitril (AIBN), oder Peroxide, beispielsweise Benzoylperoxid, oder Redoxinitiatorsysteme, wie eine Mischung von Eisen(III)acetyl-acetonat, Benzoin und Benzoylperoxid, oder photochemische Radikalbildner, wie Benzoin oder Benzilmethylketal.

Die Polymerisation wird bevorzugt in Lösung durchgeführt. Die Reaktionstemperatur bewegt sich im allgemeinen im Bereich von 10 bis 200 °C, vorzugsweise zwischen 40 und 150 °C, besonders bevorzugt zwischen 40 und 100 °C.

Gegebenenfalls anwesende Lösungsmittel müssen unter den Reaktionsbedingungen inert sein. Als Lösungsmittel kommen u.a. aromatische Kohlenwasserstoffe, chlorierte Kohlenwasserstoffe, Ketone und Ether in Betracht. Beispiele dafür sind Benzol, Toluol, Xylol, Ethylbenzol, Isopropylbenzol, Ethylenchlorid, Propylenchlorid, Methylenchlorid, Chloroform, Methylethylketon, Aceton, Cyclohexanon, Diethylether oder Tetrahydrofuran.

Wie eingangs erwähnt eignen sich die erfindungsgemässen Polymeren sehr gut als Basisharze für DUV-Photoresists mit hoher Sensitivität.

Die Erfindung betrifft somit auch strahlungsempfindliche Zusammensetzungen enthaltend
(a) ein Polymeres enthaltend, bezogen auf die Gesamtmenge der im Polymer vorhandenen Strukturelemente, 100 bis 5 mol% eines wiederkehrenden Strukturelementes der Formel (VII) und 95 bis 0 mol% eines wiederkehrenden Strukturelementes der Formel (VIII) und
(b) eine unter Einwirkung von aktinischer Strahlung säurebildende Substanz.

Als strahlungsempfindliche Komponenten (b), die bei Lichteinwirkung Säure bilden bzw. abspalten, sind eine grosse Anzahl von Verbindungen bekannt. Dazu zählen beispielsweise Diazoniumsalze, wie sie in der Diazotypie verwendet werden, o-Chinondiazide, wie sie in bekannten positiv arbeitenden Kopiermassen verwendet werden, oder auch Halogenverbindungen, die bei Bestrahlung Halogenwasserstoffsäure bilden. Verbindungen dieser Art sind beispielsweise in den US-Patenten 3,515,552, 3,536,489 oder 3,779,778, sowie in den DE-OS 2,718,259, 2,243,621 oder 2,610,842 beschrieben.

Als strahlungsempfindliche Komponenten (b) der erfindungsgemässen Zusammensetzungen eignen sich vor allem kationische Photoinitiatoren aus der Gruppe der Iodonium- oder Sulfoniumsalze. Solche Verbindungen sind sind beispielsweise in "UV-Curing, Science and Technology" (Editor: S.P. Pappas, Technology Marketing Corp., 642 Westover Road, Stanford, Connecticut, USA) beschrieben.

Ferner können Sulfoxoniumsalze als strahlungsempfindliche Verbindungen verwendet werden. Solche Salze sind beispielsweise in der EP-PS 35,969 oder in der EP-A 44,274 bzw. 54,509 beschrieben. Insbesondere zu erwähnen sind aliphatische Sulfoxoniumsalze, die im tiefen UV-Bereich absorbieren.

Es lassen sich auch Verbindungen einsetzen, die bei Bestrahlung mit aktinischem Licht Sulfonsäuren freisetzen. Solche Verbindungen sind an sich bekannt und beispielsweise in der GB-A 2,120,263, den EP-A 84,515; 37,512 oder 58,638 bzw. in den US-Patenten 4,258,121 oder 4,371,605 beschrieben.

Werden als strahlungsempfindliche säureabspaltende Komponenten (b) Salze eingesetzt, so sind diese vorzugsweise in organischen Lösungsmitteln löslich. Besonders bevorzugt handelt es sich bei diesen Produkten um Salze von komplexen Säuren, beispielsweise von Tetrafluorborsäure, Hexafluorphosphorsäure, Hexafluorarsensäure oder Hexafluorantimonsäure.

Vorzugsweise wird in den erfindungsgemässen Zusammensetzungen als Komponente (b) ein Iodonium-, Sulfonium- oder Sulfoxoniumsalz eingesetzL

Besonders bevorzugt als Komponente (b) sind Triphenylsulfoniumhexafluoroarsenat, Triphenylsulfoniumhexafluoroantimonat und Triphenylsulfoniumtrifluormethylsulfonat (Triphenylsulfoniumtriflat)

Vorzugsweise enthalten die erfindungsgemässen Zusammensetzungen, bezogen auf die Gesamtmenge der Komponenten (a) und (b), 85-99 Gew.-%, insbesondere 92-98 Gew.-%, der Komponente (a) und 1-15 Gew.-%, insbesondere 2-8 Gew.-%, der Komponente (b).

Den erfindungsgemässen strahlungsempfindlichen Gemischen können gegebenenfalls auch Bindemittel (c) zugesetzt werden, was besonders zweckmässig ist, wenn es sich bei den lichtempfindlichen Zusammensetzungen um flüssige oder niedrigviskose Gemische handelt.

Die Menge des Bindemittels (c) kann 30-90 Gew.%, vorzugsweise 60-90 Gew.%, betragen, bezogen auf die gesamte Menge an Komponenten (a), (b) und (c).

Die Wahl des Bindemittels erfolgt je nach dem Anwendungsgebiet und den hierfür geforderten Eigenschaften, wie Entwickelbarkeit in wässrigen und wässrig-alkalischen Lösungsmittelsystemen oder Adhäsion auf Substraten.

Geeignete Bindemittel (c) sind beispielsweise Novolake, die sich von einem Aldehyd, vorzugsweise Formaldehyd, Acetaldehyd oder Furfurylaldehyd, besonders jedoch von Formaldehyd, und einem Phenol ableiten. Die phenolische Komponente dieser Bindemittel ist vorzugsweise Phenol selbst, oder auch halogeniertes Phenol, beispielsweise substituiert mit ein bis zwei Chloratomen, vorzugsweise p-Chlorphenol, oder sie ist ein durch ein bis zwei C₁-C₉-Alkylgruppen substituiertes Phenol, beispielsweise o- m- oder p-Kresol, ein Xylenol, p-tert.Butylphenol oder p-Nonylphenol. Es kann sich bei der Phenolkomponente der bevorzugten Novolake aber auch um p-Phenylphenol, Resorcin, Bis-(4-hydroxyphenyl)-methan oder 2,2-Bis-(4-hydroxyphenyl)-propan handeln.

Ein Teil der phenolischen Hydroxygruppen dieser Novolake kann gegebenenfalls durch Umsetzung mit Chloressigsäure, Isocyanaten, Epoxiden oder Carbonsäureanhydriden modifiziert sein.

Weitere geeignete Bindemittel sind beispielsweise Poly(4-hydroxystyrol) oder Copolymere von Maleinsäureanhydrid mit Styrol oder Vinylethern oder 1-Alkenen. Ebenfalls als Bindemittel einsetzen lassen sich:
Homo- und copolymere Acrylate und Methacrylate, z.B. Copolymere aus Methylmethacrylat/Ethylacrylat/Methacrylsäure, Poly(methacrylsäurealkylester) oder Poly(acrylsäurealkylester), wobei Alkyl C₁-C₂₀ ist.

Vorzugsweise verwendet man als Bindemittel eine alkalilösliche Substanz, beispielsweise einen Novolak (gegebenenfalls modifiziert, wie oben beschrieben), Poly(4-hydroxystyrol), Copolymere von Maleinsäureanhydrid mit Styrol oder Vinylethern oder 1-Alkenen, sowie Copolymere von Estern der Acrylsäure oder der Methacrylsäure mit ethylenisch ungesättigten Säuren, beispielsweise Methacrylsäure oder Acrylsäure.

Diesen alkalilöslichen Bindemitteln können gegebenenfalls noch weitere Zusatzharze beigegeben werden, wie bei den Positiv-Systemen auf Diazoketon-Basis üblich. Zu diesen Zusatzharzen zählen beispielsweise Vinylpolymerisate, wie Polyvinylacetat, Polyacrylate, Polyvinylether oder Polyvinylpyrrolidone. Im allgemeinen werden jedoch nicht mehr als 20 Gew.%, bezogen auf die Menge an alkalilöslichem Bindemittel, von diesen Zusatzharzen zugefügt.

Die erfindungsgemässen Zusammensetzungen können weitere übliche Zusatzstoffe enthalten, wie z.B. Stabilisatoren, Pigmente, Farbstoffe, Füllstoffe, Haftvermittler, Verlaufmittel, Netzmittel und Weichmacher. Ferner können die Zusammensetzungen zur Applikation in geeigneten Lösungsmitteln gelöst werden.

Die erfindungsgemässen Zusammensetzungen eignen sich hervorragend als Beschichtungsmittel für Substrate aller Art, z.B. Holz, Textilien, Papier, Keramik, Glas, Kunststoffe, wie Polyester, Polyethylenterephthalat, Polyolefine oder Celluloseacetat, insbesondere in Form von Filmen, sowie Metalle, wie Al, Cu, Ni, Fe, Zn, Mg oder Co, und GaAs, Si oder SiO₂, bei denen durch bildmässiges Belichten eine Abbildung aufgebracht werden soll.

Die Herstellung der beschichteten Substrate kann z.B. erfolgen, indem man eine Lösung oder Suspension der Zusammensetzung herstellt.

Die Wahl des Lösungsmittels und die Konzentration richtet sich hauptsächlich nach der Art der Zusammensetzung und nach dem Beschichtungsverfahren. Die Lösung wird mittels bekannten Beschichtungsverfahren auf ein Substrat gleichförmig aufgebracht, z.B. durch Schleudern, Tauchen, Rakelbeschichtung, Vorhanggiessverfahren, Aufpinseln, Sprühen, speziell durch elektrostatisches Sprühen und Reverse-Rollbeschichtung. Es ist auch möglich, die lichtempfindliche Schicht auf einen temporären, flexiblen Träger zu bringen, und dann durch Schichtübertragung via Laminierung das endgültige Substrat, z.B. eine kupferkaschierte Leiterplatte, zu beschichten.

Die Auftragsmenge (Schichtdicke) und Art des Substrates (Schichtträger) sind abhängig vom gewünschten Applikationsgebiet. Besonders vorteilhaft ist es, dass die erfindungsgemässen Zusammensetzungen in weiter variablen Schichtdicken eingesetzt werden können. Dieser Schichtdickenbereich umfasst Werte von ca. 0,5 µm bis mehr als 100 µm. Mit konventionellen Positiv-Systemen auf Naphthochinondiazid-Basis sind Schichtdicken vorzugsweise kleiner als 10 µm verwendbar.

Mögliche Einsatzgebiete der erfindungsgemässen Zusammensetzungen sind die Verwendung als Photoresists für die Elektronik (Galvanoresist, Ätzresist, Lötstoppresist), die Herstellung von Druckplatten, wie Offsetdruckplatten oder Siebdruckformen, der Einsatz beim Formteilätzen oder insbesondere der Einsatz als Photoresist bei der Herstellung integrierter Schaltkreise.

Die erfindungsgemässen Photoresistzusammensetzungen zeichnen sich durch ein sehr hohes Auflösungsvermögen (Submikronbereich) aus. Da die Basisharze (a) pro Styroleinheit zwei bzw. drei säurespaltbare Gruppen enthalten, ist die Löslichkeitsdifferenz zwischen bestrahlten und unbestrahlten Bereichen und damit der zu erzielende Kontrast grösser als bei vergleichbaren bekannten Photoresist-Systemen.
Aus diesem Grund sind die erfindungsgemässen Photoresistzusammensetzungen besonders zur Herstellung von Mikrochips geeignet.
Die Verwendung der erfindungsgemässen Zusammensetzungen als Photoresist zur Herstellung von integrierten Schaltkreisen bildet daher einen weiteren Erfindungsgegenstand.

Gemäss den vielfältigen Verwendungsmöglichkeiten können die möglichen Schichtträger und die Verarbeitungsbedingungen sehr unterschiedlich sein.

Für photographische Informationsaufzeichnung dienen z.B. Folien aus Polyester, Celluloseacetat oder mit Kunststoff beschichtete Papiere; für Offsetdruckformen speziell behandeltes Aluminium, für die Herstellung gedruckter Schaltungen kupferkaschierte Laminate und für die Herstellung von integrierten Schaltkreisen Siliziumwafer. Die Schichtdicken für photographische Materialien und Offsetdruckformen betragen ca. 0,5 µm bis 10 µm; für gedruckte Schaltungen 1 bis ca. 100 µm.

Nach dem Beschichten wird das Lösungsmittel in der Regel durch Trocknen entfernt, und es resultiert eine Schicht des Photoresists auf dem Träger.

Nach der in üblicher Weise erfolgten bildmässigen Belichtung des Materials werden die belichteten Stellen des Photolackes durch Herauslösen in einem Entwickler entfernt.

Die Wahl des jeweiligen Entwicklers richtet sich nach der Art des Photolackes, insbesondere nach der Natur des verwendeten Bindemittels oder der entstehenden Photolyseprodukte. Der Entwickler kann wässrige Lösungen von Basen umfassen, denen gegebenenfalls organische Lösungsmittel oder deren Mischungen zugesetzt wurden.

Besonders bevorzugt als Entwickler werden wässrig alkalische Lösungen, wie sie auch für die Entwicklung von Naphthochinondiazidschichten eingesetzt werden. Dazu zählen insbesondere wässrige Lösungen von Alkalimetallsilikaten, -phosphaten, -hydroxiden und -carbonaten. Diesen Lösungen können gegebenenfalls noch kleinere Mengen an Netzmitteln und/oder organischen Lösungsmitteln zugesetzt sein.

Typische organische Lösungsmittel, die den Entwicklerflüssigkeiten zugesetzt werden können, sind beispielsweise Cyclohexanon, 2-Ethoxyethanol, Toluol, Aceton, sowie Mischungen zweier oder mehrerer dieser Lösungsmittel.

Der Begriff 'bildmässige' Belichtung beinhaltet sowohl die Belichtung durch eine Photomaske, die ein vorbestimmtes Muster enthält, beispielsweise ein Diapositiv, die Belichtung durch einen Laserstrahl, der beispielsweise computergesteuert über die Oberfläche des beschichteten Substrates bewegt wird, und auf diese Weise ein Bild erzeugt, die Bestrahlung mit computergesteuerten Elektronenstrahlen, sowie die Bestrahlung mit Röntgenstrahlen durch eine entsprechende Maske.

Als Strahlungsquellen können im Prinzip sämtliche Lampen verwendet werden, die Strahlung im DUV-Bereich (ca. 200-300 nm) emittieren. Es sind sowohl Punktlichtquellen als auch flächenförmige Strahler (Lampenteppiche) geeignet. Beispiele sind: Kohlelichtbogenlampen, Xenon-Lichtbogenlampen, Quecksilberdampflampen, gegebenenfalls mit Metall-Halogeniden dotiert (Metall-Halogenlampen), Fluoreszenzlampen, Argonglühlampen, Elektronenblitzlampen, photographische Flutlichtlampen, Elektronenstrahlen und Röntgenstrahlen. Der Abstand zwischen Lampe und erfindungsgemässem Bildmaterial kann je nach Anwendungszweck und Lampentyp bzw. -stärke variieren, z.B. zwischen 2 cm bis 150 cm. Speziell geeignet sind Laserlichtquellen, z.B. Argonionenlaser oder Kryptonionenlaser. Mit Laserlicht kann der Resist auch ohne Maske belichtet werden, indem der gesteuerte Laser-Strahl direkt auf der Resist-Schicht schreibt. Hier ist die hohe Empfindlichkeit der erfindungsgemässen Materialien sehr vorteilhaft, die hohe Schreibgeschwindigkeiten bei relativ niedrigen Intensitäten erlaubt. Nach dieser Methode können gedruckte Schaltungen in der Elektronikindustrie, lithographische Offsetdruckplatten oder Reliefdruckplatten sowie photographische Bildaufzeichnungsmaterialien hergestellt werden. Die hohe Empfindlichkeit der Resists ist auch für die Belichtung mittels DUV-Stepper von Vorteil, da sehr kurze Belichtungszeiten erwünscht sind.

### Beispiele

### I. Herstellung der Monomeren

### I.1. 3,4-Di(2-tetrahydropyranyloxy)styrol

### a) Herstellung von 3,4-Dihydroxystyrol

200 g (1,11 mol) Kaffeesäure werden in 1,2 l Dimethylformamid gelöst und 3 h bei 150 °C gerührt. Nach Abkühlen auf Raumtemperatur wird die Lösung auf 3 kg Eis gegossen, mit Natriumchlorid gesättigt und zweimal mit Essigester extrahiert. Die organische Phase wird mit 2%iger NaHCO₃-Lösung und anschliessend mit Wasser gewaschen und nach Trocknen über Na₂SO₄ eingedampft. Es werden 120 g (80%) einer hochviskosen Flüssigkeit erhalten, die aus Toluol umkristallisiert werden kann. Die Substanz weist jedoch eine genügend hohe Reinheit auf und kann ohne Umkristallisation weiterverarbeitet werden.

| Elementaranalyse: | | |
|---|---|---|
| Ber. = | C: 70,52 | H: 5,97 |
| Gef. = | C: 70,25 | H: 6,02 |

¹H-NMR (CDCl₃/DMSO):
δ = 4,99; 5,03; 5,44; 5,51 ppm (dd, 2H)
δ = 6,47; 6,50; 6,53; 6,56 ppm (m, 1H)
δ 6,66-6,74; 6,89 (m, 3H)
δ = 8,53 ppm (s, 1H)
δ = 8,63 ppm (s, 1H)

### b) Herstellung von 3,4-Di(2-tetrahydropyranyloxy)styrol

100 g (0,73 mol) des unter a) hergestellten 3,4-Dihydroxystyrols werden mit 320 g (3,8 mol) Dihydropyran und einigen Tropfen konzentrierter Salzsäure versetzt. Das Reaktionsgemisch wird 12 h bei 40 °C gerührt. Anschliessend wird die Lösung mit Diethylether verdünnt und auf Eis gegossen. Die organische Phase wird zweimal bei 0 °C mit 1N-NaOH gewaschen, getrocknet und mit Aktivkohle versetzt. Nach Filtrieren und Eindampfen des Filtrats wird eine farblose viskose Flüssigkeit erhalten, die über eine Kieselgelsäule mit einem Hexan/Essigester-Gemisch-Gemisch (4:1) chromatographiert wird.
Ausbeute: 111 g (50 %)

| Elementaranalyse: | | |
|---|---|---|
| Ber. = | C: 71,03 | H: 7,95 |
| Gef. = | C: 70,86 | H: 8,18 |

¹H-NMR (CDCl₃):
δ = 1,62-2,05 ppm (m, 12H)
δ = 3,57-3,63 ppm und 3,86-4,12 ppm (m, 4H)
δ = 5,11-5;14 ppm und 5,57-5,63 ppm (m, 2H)
δ = 5,41-5,46 ppm (m, 2H)
δ = 6,57-6,66 ppm (m, 1H)
δ = 6,97-7,24 ppm (m, 3H)

### I.2. 3,4-Di(2-tetrahydrofuranyloxy)styrol

38,1 g (0,28 mol) 3,4-Dihydroxystyrol und 118 g (1,68 mol) 2,3-Dihydrofuran werden mit 5 Tropfen konzentrierter Salzsäure versetzt, wobei sofort eine exotherme Reaktion einsetzt. Die Lösung wird 4 h bei 50 °C gerührt und anschliessend auf ein Gemisch aus Eiswasser und Diethylether gegossen. Nach zweimaligem Extrahieren mit Diethylether wird die organische Phase bei 0 °C dreimal mit 1N-NaOH gewaschen und über Na₂SO₄ getrocknet. Nach Abdampfen des Lösungsmittels wird eine klare Flüssigkeit erhalten, welche mittels Kugelrohrdestillation (155 °C/0,02 Torr) oder säulenchromatographisch (Kieselgel, Hexan/Essigester 4:1) gereinigt wird.
Ausbeute: 47 g (61 %)

| Elementaranalyse: | | |
|---|---|---|
| Ber. = | C: 69,54 | H: 7,30 |
| Gef. = | C: 69,36 | H: 7,44 |

¹H-NMR (CDCl₃):
δ = 1,84-2,23 ppm (m, 8H)
δ = 3,89-4,12 ppm (m, 4H)
δ = 5,10; 5,15 ppm und 5,58; 5,64 ppm (m, 2H)
δ = 5,75 ppm (m, 2H)
δ = 6,57-6,66 ppm (m, 1H)
δ = 6,97-7,24 ppm (m, 3H)

### I.3. 3,4-Di(t-butoxycarbonyloxy)styrol

38 g (0,28 mol) 3,4-Dihydroxystyrol werden in 1 l Tetrahydrofuran gelöst und auf 5 °C gekühlt. Innerhalb von 20 min werden unter Rühren und Kühlen 63 g (0,56 mol) Kalium-tert.-butylat portionsweise hinzugegeben, so dass die Temperatur der Mischung zwischen 5 °C und 8 °C bleibt. Das Gemisch wird 1 h bei Raumtemperatur gerührt. Anschliessend wird eine Lösung von 135,3 g (0,62 mol) Di-tert.-butylcarbonat in wenig THF zugetropft. Nach ca. 3 h Rühren bei Raumtemperatur zeigt eine dünnschichtchromatographische Analyse (Hexan/Essigester 4:1) das Ende der Reaktion an. Das Reaktionsgemisch wird auf 2 kg Eis gegossen und zweimal mit Diethylether extrahiert. Die organische Phase wird je zweimal mit 1N-NaOH und anschliessend mit Wasser gewaschen und getrocknet. Anschliessend wird das Lösungsmittel am Rotationsverdampfer abdestilliert. Der viskose Rückstand wird säulenchromatographisch (Kieselgel, Hexan/Essigester 4:1) gereinigt.
Ausbeute: 23g (24 %)

| Elementaranalyse: | | |
|---|---|---|
| Ber. = | C:64,27 | H:7,19 |
| Gef. = | C: 63,90 | H: 7,03 |

¹H-NMR (CDCl₃):
δ = 1,52-1,56 ppm (m, 18H)
δ = 5,24; 5,28 ppm und 5,66; 5,72 ppm (m, 2H)
δ = 6,60-6,70 ppm (m, 1H)
δ = 7,19-7,30 ppm (m, 3H)

### I.4. 3,5-Di(2-tetrahydropyranyloxy)styrol

### a) Herstellung von 3,5-Diacetoxybenzoesäure:

50 g (0,32 mol) 3,5-Dihydroxybenzoesäure werden in 100 ml Essigsäureanhydrid vorgelegt. Unter Kühlung wird eine Lösung aus 10 Tropfen konzentrierter Schwefelsäure in 60 ml Essigsäureanhydrid langsam zugetropft, so dass die Innentemperatur zwischen 20 °C und 25 °C gehalten wird. Das Reaktionsgemisch wird 1 h bei Raumtemperatur gerührt und anschliessend auf Eiswasser gegossen, wobei das Produkt ausfällt. Das Rohprodukt wird getrocknet und aus Toluol umkristallisiert.
Ausbeute: 52 g (57 %)
Schmelzpunkt: 160,4 °C

| Elementaranalyse: | | |
|---|---|---|
| Ber. = | C: 55,47 | H: 4,23 |
| Gef. = | C: 55,56 | H: 4,24 |

¹H-NMR (CD₃OD):
δ= 2,28 ppm (s, 6H)
δ = 4,91 ppm (s, 1H)
δ = 7,18-7,20 ppm und 7,62-7,64 ppm (m, 3H)

### b) Herstellung von 3,5-Diacetoxybenzoesäurechlorid:

833 g (3,5 mol) der unter a) hergestellten 3,5-Diacetoxybenzoesäure, 1 kg (8,4 mol) Thionylchlorid und 3 ml Dimethylformamid werden in 5,3 l Essigester gelöst und auf 70 °C erhitzt. Die Lösung wird bei dieser Temperatur so lange gerührt, bis keine HCl-bzw. SO₂-Entwicklung mehr festzustellen ist. Nach Abdampfen des Lösungsmittels wird das so erhaltene Rohprodukt aus Cyclohexan umkristallisiert.
Ausbeute: 785 g (87 %)
Schmelzpunkt: 90 °C

| Elementaranalyse: | | | |
|---|---|---|---|
| Ber. = | C: 51,48 | H: 3,53 | Cl: 13,81 |
| Gef. = | C: 51,45 | H: 3,68 | Cl: 13,70 |

¹H-NMR (CDCl₃):
δ = 2,31 ppm (s, 6H)
δ = 7,27-7,29 ppm und 7,72-7,77 ppm (m, 3H)

### c) Herstellung von 3,5-Diacetoxystyrol:

170 g (0,66 mol) des nach b) hergestellten 3,5-Diacetoxybenzoesäurechlorids, 89,6 g (0,66 mol) N-Benzyldimethylanilin, und 1,5 g (6,6 mmol) Palladium(II)acetat werden in 1,7 l Toluol gelöst. In einem 3l-Autoklaven wird diese Lösung 10 h bei 95 °C mit Ethylen unter einem Druck von 10 Atmosphären umgesetzt. Danach wird die Reaktionslösung mit 1N-HCL, mit 1N-NaOH und anschliessend mit Wasser gewaschen. Nach Trocknen über Na₂SO₄ wird die organische Phase mit Aktivkohle entfärbt und filtriert. Durch Eindampfen des Filtrats werden 120 g (0,54 mol = 82%) einer viskosen Flüssigkeit erhalten, die ohne Reinigung weiterverarbeitet werden kann.
Für analytische Zwecke kann die Substanz säulenchromatographisch (Kieselgel, Hexan/Essigester 2:1) oder mittels Kugelrohrdestillation (140 °C/0,02 Torr) gereinigt werden.

| Elementaranalyse: | | |
|---|---|---|
| Ber. = | C: 65,45 | H: 5,49 |
| Gef. = | C: 65,42 | H: 5,56 |

¹H-NMR (CDCl₃):
δ = 2,24 ppm (s, 6H)
δ = 5,28; 5,32 ppm und 5,6 9; 5,75 ppm (m, 2H)
δ = 6,58-6,68 ppm (m, 1H)
δ = 6,81-7,00 ppm (m, 3H)

### d) Herstellung von 3,5-Dihydroxystyrol:

Zu einer Lösung von 20 g (357mmol) Kaliumhydroxid in 200 ml Methanol werden unter Rühren und Eiskühlung 20 g (91 mmol) 3,5-Diacetoxystyrol so langsam hinzugegeben, dass eine Reaktionstemperatur von 30 °C nicht überschritten wird Nach einigen Minuten wird mittels Dünnschichtchromatographie festgestellt, dass kein Edukt mehr vorhanden ist. Das Reaktionsgemisch wird auf Eiswasser gegossen und mit Ether gewaschen. Die wässrige Phase wird bei 0 °C mit Schwefelsäure bis zum pH1 angesäuert. Nach zweimaliger Extraktion mit Ether, Trocknen der organischen Phase und Eindampfen des Lösungsmittels werden 10 g (80 %) eines Feststoffs erhalten, der sich aus Toluol umkristallisieren lässt.
Schmelzpunkt: 84 °C

| Elementaranalyse: | | |
|---|---|---|
| Ber. = | C: 70,58 | H: 5,92 |
| Gef. = | C: 70,26 | H: 5,93 |

¹H-NMR (Aceton-d₆):
δ = 5,13; 5,17 ppm und 5,63; 5,69 ppm (m, 2H)
δ = 6,30; 6,44 ppm (m, 3H)
δ = 6,54-6,63 ppm (m, 1H)
δ = 8,2 ppm (s, 2H)

### e) Herstellung von 3,5-Di(2-tetrahydropyranyloxy)styrol:

Eine Lösung von 80 g ( 587 mmol) 3,5-Dihydroxystyrol in 300 g Dihydropyran wird mit 0,5 ml konzentrierter Salzsäure versetzt und 2 h bei 40 °C gerührt. Anschliessend wird die Mischung auf Eiswasser gegossen und zweimal mit Ether extrahiert. Die organische Phase wird bei 0 °C dreimal mit 1N-NaOH gewaschen, getrocknet und eingeengt. Der Rückstand wird säulenchromatographisch (Kieselgel, Hexan/Essigester 4:1) gereinigt, wobei 110 g (62 %) einer viskosen Flüssigkeit erhalten werden.

| Elementaranalyse: | | |
|---|---|---|
| Ber. = | C: 71,03 | H: 7,95 |
| Gef. = | C: 70,96 | H: 8,03 |

¹H-NMR (CDCl₃):
δ = 1,54-2,04 ppm (m, 12H)
δ = 3,52-3,95 ppm (m, 4H)
δ = 5,19; 5,23 ppm und 5,67; 5,73 ppm (m, 2H)
δ = 5,39-5,43 ppm (m, 2H)
δ = 6,57-6,67 ppm (m, 1H)
δ = 6,70-6,76 ppm (m, 3H)

### I.5. 3.5-Di(2-tetrahydrofuranyloxy)styrol

Eine Mischung aus 45 g (330 mmol) 3,5-Dihydroxystyrol und 140 g Dihydrofuran wird mit 5 Tropfen konzentrierter Salzsäure versetzt, wobei sofort eine exotherme Reaktion einsetzt. Das Gemisch wird ca. 3 h bei 40-45 °C gerührt und anschliessend auf Eiswasser gegossen. Nach zweimaliger Extraktion mit Ether wird die organische Phase dreimal mit 1N-NaOH gewaschen, getrocknet, mit Aktivkohle versetzt und filtriert. Nach Eindampfen des Lösungsmittels wird eine klare viskose Flüssigkeit erhalten, die säulenchromatographisch (Kieselgel, Hexan/Essigester 4:1) gereinigt wird.
Ausbeute: 40 g (44 %)

| Elementaranalyse: | | |
|---|---|---|
| Ber. = | C: 69,55 | H: 7,30 |
| Gef. = | C: 69,07 | H: 7,59 |

¹H-NMR (CDCl₃):
δ = 1,84-2,17 ppm (m, 8H)
δ = 3,82-4,07 ppm (m, 4H)
δ = 5,19; 5,23 ppm und 5,67; 5,73 ppm (m, 2H)
δ = 5,77-5,80 ppm (m, 2H)
δ = 6,56-6,64 ppm (m, 1H)
δ = 6,64-6,73 ppm (m, 3H)

### I.6. 3,5-Di(tert.-butyloxycarbonyloxy)styrol

Unter Stickstoff wird eine Lösung von 43 g (320 mmol) 3,5-Dihydroxystyrol in 1 l THF vorgelegt und auf 5 °C abgekühlt. Innerhalb von 5 Minuten werden 72 g Kalium-t-butylat so langsam zugegeben, dass eine Innentemperatur von 7 °C nicht überschritten wird. Das Gemisch wird dann 1 h bei Raumtemperatur gerührt. Anschliessend werden 153 g (700 mmol) Di-t-butylcarbonat zugegeben. Nach 2 h Rühren bei Raumtemperatur wird das Gemisch auf Eis gegossen und zweimal mit Ether extrahiert. Die organische Phase wird zweimal mit 1N-NaOH und zweimal mit Wasser gewaschen, über Na₂SO₄ getrocknet und eingedampft. Der Rückstand wird aus Hexan umkristallisiert, wobei 40 g (37 %) einer kristallinen Substanz mit dem Schmelzpunkt 69 °C erhalten werden.

| Elementaranalyse: | | |
|---|---|---|
| Ber. = | C: 64,27 | H: 7,19 |
| Gef. = | C: 64,20 | H: 7,31 |

¹H-NMR (CDCl₃):
δ = 1,55 ppm (s, 18H)
δ = 5,29; 5,33 ppm und 5,71; 5,77 ppm (m, 2H)
δ = 6,60-6,69 ppm (m, 1H)
δ = 6,97-7,09 ppm (m, 3H)

### II. Herstellung der Polymeren

### II.1. Poly[3,4-di(2-tetrahydropyranyloxy)styrol]

Eine Lösung von 120 g (395 mmol) 3,4-Di(2-tetrahydropyranyloxy)styrol in 310 g Toluol wird mit 0,65 g Azobisisobutyronitril versetzt. An einer Vakuum/Stickstoff-Linie wird die Lösung von Sauerstoff befreit. Das Gemisch wird unter Stickstoff 16 h bei 70 °C gerührt. Anschliessend wird das Polymere durch Eingiessen in n-Hexan gefällt und aus THF/n-Hexan umgefällt. Nach dem Trocknen im Hochvakuum werden 77 g (63 %) eines weissen Pulvers erhalten.

| Elementaranalyse: | | |
|---|---|---|
| Ber. = | C: 71,03 | H: 7,95 |
| Gef. = | C: 70,83 | H: 8,06 |

Die TGA-Analyse zeigt einen Gewichtsverlust von ca. 56 % oberhalb 220 °C, was auf die thermische Abspaltung von Dihydropyran hinweist.
Die DSC-Analyse zeigt einen endothermen Peak bei 261 °C, was ebenfalls auf die Abspaltung von Dihydropyran zurückzuführen ist.
Mittels Gelpermeationschromatographie (GPC) in THF wird ein Molekulargewicht Mₙ=20000 bzw. M_{w}=61000 sowie eine Molekulargewichtsverteilung von 3 gemessen.

UV-Absorption: ein 1 µm dicker Film des Polymeren auf Quarz zeigt bei 250 nm eine Absorption von 0,12. Das Polymere ist daher sehr gut zur Anwendung in der DUV-Lithographie geeignet.

### II.2. Poly[3,5-di(tetrahydropyranyloxy)styrol]

Analog zu Beispiel II.1. werden 8,5 g (28 mmol) 3,5-Di(2-tetrahydropyranyloxy)styrol polymerisiert.
Ausbeute: 5 g (59 %)

| Elementaranalyse: | | |
|---|---|---|
| Ber. = | C: 71,03 | H: 7,95 |
| Gef. = | C: 70,89 | H: 8,03 |

TGA: im Temperaturbereich von 190-240 °C ist eine Gewichtsabnahme von ca. 56 % zu beobachten. Nach der Abspaltung von Dihydropyran ist das Polymere bis über 400 °C stabil.

DSC: endothermer Peak bei 230 °C (Abspaltung von 2 Dihydropyranmolekülen pro Monomermolekül).

GPC (THF):
Mₙ = 18500
M_{w} = 57700
Molekulargewichtsverteilung: 3,1

UV-Absorption: ein 1 µm dicker Film des Polymeren auf Quarz zeigt bei 248 nm eine Absorption von 0,1.

### II.3. Poly[3,4-di(t-butyloxycarbonyloxy)styrol]

Analog zu Beispiel II.1. werden 22 g (65 mmol) 3,4-Di(t-butyloxycarbonyloxy)styrol polymerisiert.
Ausbeute: 8,5 g (40 %)

| Elementaranalyse: | | |
|---|---|---|
| Ber. = | C: 64,27 | H: 7,19 |
| Gef. = | C: 64,24 | H: 7,21 |

TGA: im Temperaturbereich von 150-180 °C ist eine Gewichtsabnahme von ca. 60 % zu beobachten (Abspaltung der beiden t-Butoxycarbonylgruppen).

DSC: scharfer endothermer Peak bei 176 °C

GPC (THF):
Mₙ = 25000
M_{w} = 102000
Molekulargewichtsverteilung: 4

UV-Absorption: ein 1 µm dicker Film des Polymeren auf Quarz zeigt im Bereich von 235-255 nm eine Absorption von 0,1.

### II.4. Poly[3,5-di(t-butoxycarbonyloxy)styrol]

Analog zu Beispiel II.1. werden 35 g (104 mmol) 3,5-Di(t-butoxycarbonyloxy)styrol polymerisiert.
Ausbeute: 15 g (43 %)

| Elementaranalyse: | | |
|---|---|---|
| Ber. = | C: 64,27 | H: 7,19 |
| Gef. = | C: 64,24 | H: 7,23 |

TGA: ab 160 °C ist eine scharfe Gewichtsabnahme von ca. 60 % zu beobachten (Abspaltung der beiden t-Butoxycarbonylgruppen).
DSC: scharfer endothermer Peak bei 174 °C
GPC (THF):
Mₙ = 20000
M_{w} = 70000
Molekulargewichtsverteilung: 3,5

UV-Absorption: ein 1 µm dicker Film des Polymeren auf Quarz zeigt im Bereich von 235-255 nm eine Absorption von 0,1.

### III. Anwendungsbeispiele

III.1. 24 g Poly[3,4-di(2-tetrahydropyranyloxy)styrol] und 1,2 g Triphenylsulfoniumhexafluoroantimonat werden in 170 g Cyclopentanon gelöst. Diese Lösung wird durch ein 0,5 µm-Filter filtriert und auf eine Siliziumscheibe gebracht. Durch Schleuderbeschichtung mit 2000 U/min während 20 s wird ein homogener Film auf dem Siliziumwafer erzeugt, der anschliessend 1min bei 90 °C getrocknet wird. Die Schichtdicke des so erhaltenen Films beträgt 0,9 µm.
   Der Film wird durch eine Chrom/Quarz-Maske mit Strahlung der Wellenlänge 254 nm belichtet. Zur vollständigen Belichtung ist eine Strahlungsdosis von 1-3 mJ/cm² erforderlich. Nach der Belichtung wird der Wafer auf einer Heizplatte 10-30 s einer Temperatur von 90 °C ausgesetzt. Der belichtete Film wird dann 30 bis 60 Sekunden lang mit einem metallionenfreien wässrig-alkalischen Entwickler (Microposit MF 312) entwickelt, wobei die belichteten Zonen herausgelöst werden. Nach dem Spülen mit deionisiertem Wasser wird der Resist eine Minute lang bei 100 °C getrocknet. Aufnahmen der Resiststrukturen im Rasterelektronenmikroskop zeigen, dass Strukturen von 0,5 µm gut aufgelöst sind.
III.2. Analog zu Beispiel III.1. wird ein Photoresistfilm aus einer Lösung von 2 g Poly[3,4-di(t-butoxycarbonyloxy)styrol] und 0,1 g Triphenylsulfoniumhexafluoroarsenat in 12 g Cyclopentanon hergestellt (Schleuderbeschichtung: 4000 U/min während 20 s, Trocknung: 2 min/120 °C, Schichtdicke: 0,95 µm). Der Resistfilm wird wie in Beispiel III.1. belichtet (254 nm, 3-4 mJ/cm², thermische Nachbehandlung: 30 s/110 °C) und entwickelt (Selectiplast PD 2007, mit Wasser auf das doppelte Volumen verdünnt). Strukturen von 0,5 µm sind aufgelöst.
III.3. Analog zu Beispiel III.1. wird ein Photoresistfilm aus einer Lösung von 5 g Poly[3,5-di(t-butoxycarbonyloxy)styrol] und 0,25 g Triphenylsulfoniumhexafluoroarsenat in 25 g Cyclopentanon hergestellt (Schleuderbeschichtung: 3500 U/min, Trocknung: 2 min/90 °C, Schichtdicke: 1,2 µm). Der Resistfilm wird wie in Beispiel III.1. belichtet (254 nm, 4-6 mJ/cm²) und entwickelt (Microposit 312 der Firma Shipley). Strukturen von 0,5 µm sind aufgelöst.
III.4. Analog zu Beispiel III.1. wird ein Photoresistfilm aus einer Lösung von 2 g Poly[3,5-di(2-tetrahydropyranyloxy)styrol] und 0,1 g Triphenylsulfoniumtriflat in 12 g Cyclopentanon hergestellt (Schleuderbeschichtung: 3000 U/min, Trocknung: 2 min/90 °C, Schichtdicke: 1,05 µm). Der Resistfilm wird wie in Beispiel III.1. belichtet (254 nm, 10 mJ/cm², thermische Nachbehandlung: 2 min/90 °C) und entwickelt (0,5-N-NaOH). Strukturen von 0,5 µm sind aufgelöst.

## Patentansprüche

1. Verbindungen der Formel(I) 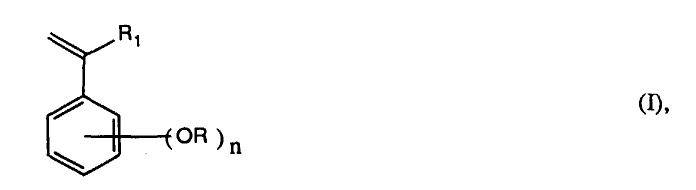 worin R₁ für Wasserstoff, Methyl oder Halogen steht, n 2 oder 3 bedeutet und R für C₁-C₆-Alkyl, Benzyl, 2-Tetrahydrofuranyl, 2-Tetrahydropyranyl, C₁-C₆-Alkoxycarbonyl, Phenoxycarbonyl oder Benzyloxycarbonyl steht, oder, falls zwei der Substituenten OR in ortho-Stellung zueinander stehen, zwei Reste R zusammen eine Ethylengruppe, die gegebenenfalls durch bis zu vier C₁-C₆-Alkylgruppen substituiert ist, oder eine C₂-C₆-Alkylidengruppe bilden.

2. Verbindungen der Formel (I) nach Anspruch 1, worin R₁ Wasserstoff ist, n 2 bedeutet und die beiden Substituenten OR in 3,4- oder 3,5-Stellung zur Vinylgruppe stehen.

3. Verbindungen der Formel (I) nach Anspruch 2, worin R tert.-Butoxycarbonyl, 2-Tetrahydrofuranyl oder 2-Tetrahydropyranyl bedeutet.

4. Polymere mit einem Molekulargewicht M_{w} von 10³ bis 10⁶ (gemessen mittels Gelpermeationschromatographie) enthaltend, bezogen auf die Gesamtmenge der im Polymer vorhandenen Strukturelemente, 100 bis 5 mol% eines wiederkehrenden Strukturelementes der Formel (VII) 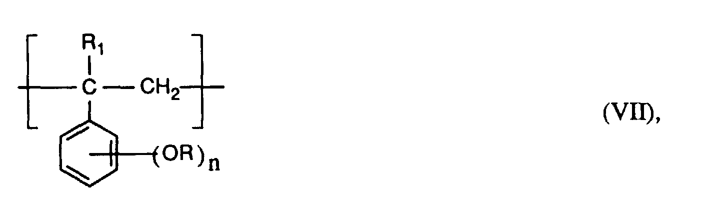 worin R₁, R und n die in Anspruch 1 angegebene Bedeutung haben, und 95 bis 0 mol% eines wiederkehrenden Strukturelementes der Formel (VIII) 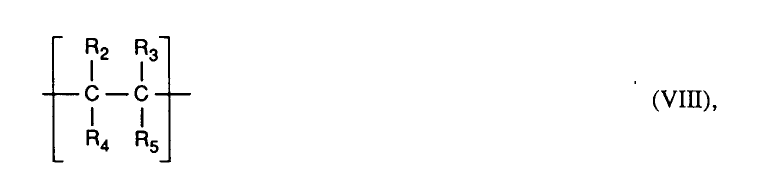 worin R₂ und R₃ unabhängig voneinander jeweils Wasserstoff, unsubstituiertes oder durch Halogenatome, Cyano- oder Nitrogruppen substituiertes C₁-C₄-Alkyl oder ein unsubstituiertes oder durch Halogenatome, C₁-C₄-Alkoxy-, Hydroxy-, Cyano- oder Nitrogruppen substituiertes Phenyl oder Naphthyl bedeuten, und R₄ und R₅ unabhängig voneinander je ein Wasserstoff- oder Halogenatom, unsubstituiertes oder durch Halogenatome, Cyano- oder Nitrogruppen substituiertes C₁-C₁₂-Alkyl, unsubstituiertes oder durch Halogenatome, Hydroxy-, Cyano-, Nitrogruppen, C₁-C₄-Alkyl oder C₁-C₄-Alkoxy substituiertes Phenyl, Naphthyl oder Benzyl oder einen der folgenden Reste -OR₆, -COOR₇, -COR₈ oder zusammen einen zweiwertigen Rest der Formeln (IX)-(XI) bedeuten, 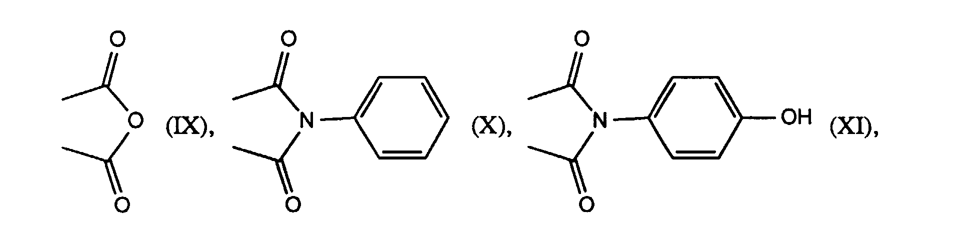 wobei R₆ und R₇ unabhängig voneinander je ein Wasserstoffatom, unsubstituiertes oder durch Halogenatome, Cyano- oder Nitrogruppen substituiertes C₁-C₁₂-Alkyl, unsubstituiertes oder durch Halogen, Cyano-, Nitrogruppen, C₁-C₄-Alkyl oder C₁-C₄-Alkoxy substituiertes Phenyl oder Naphthyl bedeuten und R₈ die gleiche Bedeutung wie R₆ hat oder für den Rest 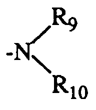 steht, worin R₉ und R₁₀ unabhängig voneinander die gleiche Bedeutung wie R₈ haben.

5. Polymere nach Anspruch 4 enthaltend 100-20 mol% eines wiederkehrenden Strukturelements der Formel (VII) und 80-0 mol% eines wiederkehrenden Strukturelements der Formel (VIII).

6. Polymere nach Anspruch 4, worin in der Formel (VII) R₁ Wasserstoff ist, n 2 bedeutet und die beiden Substituenten OR in 3,4- oder 3,5-Stellung zur Vinylgruppe stehen.

7. Polymere nach Anspruch 4, worin in der Formel (VII) R tert.-Butoxycarbonyl, 2-Tetrahydrofuranyl oder 2-Tetrahydropyranyl bedeutet.

8. Verfahren zur Herstellung von Polymeren gemäss Anspruch 4, dadurch gekennzeichnet, dass man eine Verbindung der Formel (I) gemäss Anspruch 1 oder ein Gemisch aus einer Verbindung der Formel (I) und darin bis zu 95 mol% enthaltenen Verbindungen der Formel (XII) 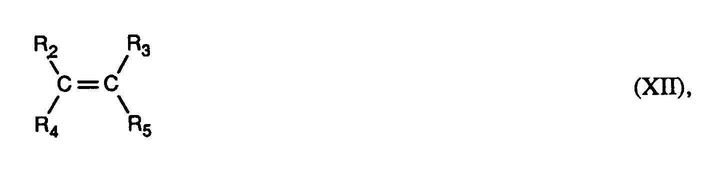 worin R₂, R₃, R₄ und R₅ die in Anspruch 4 angegebene Bedeutung haben, radikalisch polymerisiert.

9. Strahlungsempfindliche Zusammensetzung enthaltend
(a) ein Polymeres gemäss Anspruch 4 und
(b) eine unter Einwirkung von aktinischer Strahlung säurebildende Substanz.

10. Zusammensetzung nach Anspruch 9 enthaltend als Komponente (b) ein Iodonium-, Sulfonium- oder Sulfoxoniumsalz.

11. Zusammensetzung nach Anspruch 9 enthaltend als Komponente (b) Triphenylsulfoniumhexafluoroarsenat, Triphenylsulfoniumhexafluoroantimonat oder Triphenylsulfoniumtrifluorsulfonat.

12. Verwendung der Zusammensetzung nach Anspruch 9 als Photoresist zur Herstellung von integrierten Schaltkreisen.

## Claims

1. A compound of formula (I) 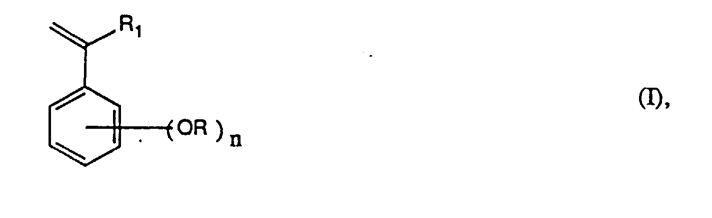 wherein R₁ is hydrogen, methyl or halogen, n is 2 or 3, and R is C₁-C₆alkyl, benzyl, 2-tetrahydrofuranyl, 2-tetrahydropyranyl, C₁-C₆alkoxycarbonyl, phenoxycarbonyl or benzyloxycarbonyl, or, if two substituents OR are ortho-positioned to each other, two substituents R together form an ethylene group which may be substituted by up to four C₁-C₆alkyl groups, or form a C₂-C₆alkylidene group.

2. A compound of formula (I) according to claim 1, wherein R₁ is hydrogen, n is 2 and both substituents OR are in 3,4- or 3,5-position to the vinyl group.

3. A compound of formula (I) according to claim 2, wherein R is tert-butoxycarbonyl, 2-tetrahydrofuranyl or 2-tetrahydropyranyl.

4. A polymer having a molecular weight M_{w} of 10³ to 10⁶ (determined by gel permeation chromatography) and containing, based on the total amount of structural units present in the polymer, 100 to 5 mol % of a structural repeating unit of formula (VII) 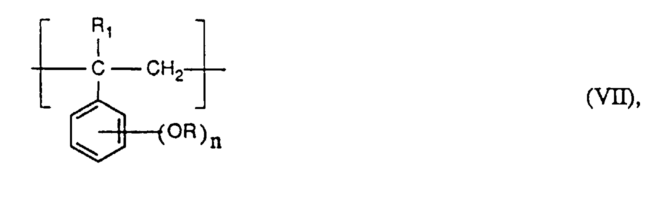 wherein R₁, R and n are as defined in claim 1,
and 95 to 0 mol % of a structural repeating unit of formula (VIII) 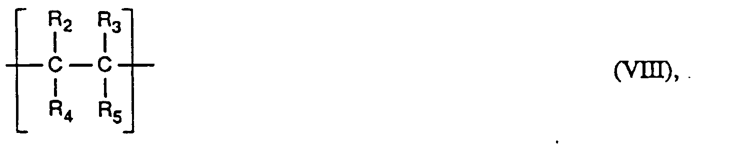 wherein R₂ and R₃ are each independently of the other hydrogen, C₁-C₄alkyl which is unsubstituted or substituted by halogen, cyano or nitro, or are phenyl or naphthyl which are unsubstituted or substituted by halogen C₁-C₄alkoxy, hydroxy, cyano or nitro, and R₄ and R₅ are each independently of the other a hydrogen or halogen atom, C₁-C₁₂alkyl which is unsubstituted or substituted by halogen atoms, cyano or nitro, or are phenyl, naphthyl or benzyl which are unsubstituted or substituted by halogen, cyano or nitro, C₁-C₄alkyl or C₁-C₄alkoxy, or are a radical selected from the group consisting of -OR₆, -COOR₇ and -COR₈ or, together with a divalent radical of formulae (IX-XI) are 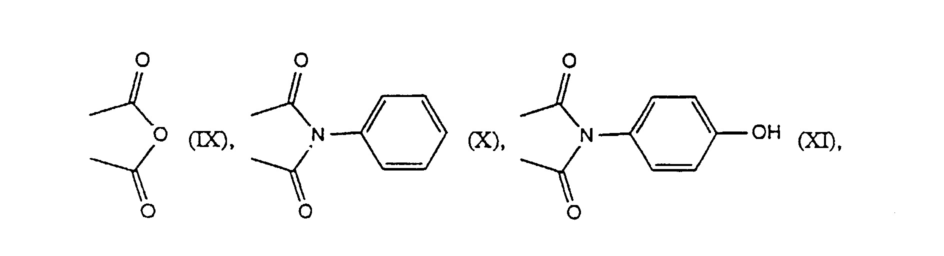 wherein R₆ and R₇ are each independently of the other hydrogen, C₁-C₁₂alkyl which is unsubstituted or substituted by halogen, cyano or nitro, or are phenyl or naphthyl, unsubstituted or substituted by halogen, cyano, nitro, C₁-C₄alkyl or C₁-C₄alkoxy, and R₈ has the same meaning as R₆ or is the radical 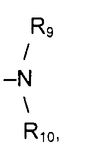 wherein R₉ and R₁₀ have each independently of the other the same meaning as R₈.

5. A polymer according to claim 4 containing 100 to 20 mol % of a structural repeating unit of formula (VII) and 80 to 0 mol % of a structural repeating unit of formula (VIII).

6. A polymer according to claim 4 containing a structural repeating unit of formula (VII), wherein R₁ is hydrogen, n is 2 and both substitutents OR are in 3,4- or 3,5-position to the vinyl group.

7. A polymer according to claim 4, wherein R in formula (VII) is tert-butoxycarbonyl, 2-tetrahydrofuranyl or 2-tetrahydropyranyl.

8. A process for the preparation of a polymer according to claim 4, which comprises subjecting a compound of formula (I) according to claim 1 or a mixture of a compound of formula (I) and compounds of formula (XII) 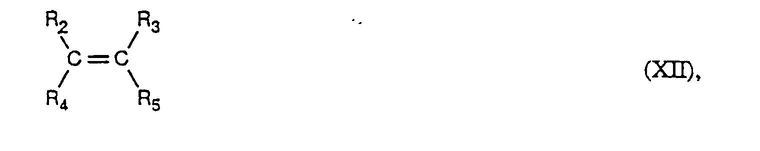 present therein in an amount of up to 95 mol %, wherein R₂, R₃, R₄ and R₅ are as defined in claim 4, to radical polymerization.

9. A radiation-sensitive composition comprising
(a) a polymer as claimed in claim 4, and
(b) a substance that generates an acid upon exposure to actinic radiation.

10. A composition according to claim 9, wherein component (b) is an iodonium-, sulfonium- or sulfoxonium salt.

11. A composition according to claim 9, wherein component (b) is selected from the group consisting of triphenylsulfoniumhexafluoroarsenate, triphenylsulfonium hexafluoroantimonate or triphenylsulfonium trifluorosulfonate.

12. Use of a composition as claimed in claim 9 as photoresist for making integrated circuits.

## Revendications

1. Un composé de la formule (I) 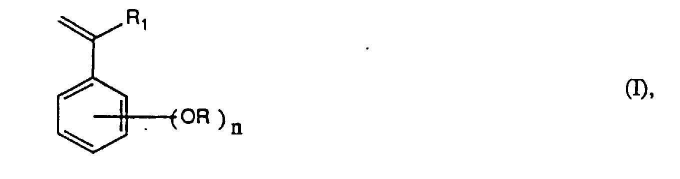 où R₁ est de l'hydrogène, du méthyle ou de l'halogène, n est 2 ou 3 et R est C₁-C₆ alkyle, benzyle, 2-tétrahydrofuranyle, 2-tétrahydropyranyle, C₁-C₆ alkoxycarbonyle, phénoxycarbonyle ou benzyloxycarbonyle ou, si deux substituants OR sont ortho-positionnés l'un par rapport à l'autre, deux substituants R ensemble forment un groupe d'éthylène qui peut être remplacé par un maximum de quatre groupes C₁-C₆ alkyle ou former un groupe C₂-C₆ alkylidène.

2. Un composé de la formule (I) conformément à la revendication 1, où R₁ est de l'hydrogène, n est 2 et les deux substituants OR sont dans la position 3,4- ou 3,5- par rapport au groupe vinyle.

3. Un composé de la formule (I) conformément à la revendication 2, où R est tert-butoxycarbonyle, 2-tétrahydrofuranyle ou 2-tétrahydropyranyle.

4. Un polymère ayant une masse moléculaire M_{w} de 10³ à 10⁶ (déterminée par chromatographie d'exclusion diffusion) et contenant, sur la base du montant total des unités structurales présentes dans le polymère, de 100 à 5 moles % d'une unité répétitive structurale de la formule (VII) 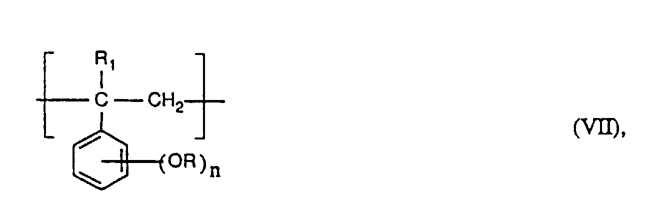 où les valeurs R₁, R et n sont définies dans la revendication 1,
et de 95 à 0 moles % d'une unité répétitive structurale de la formule (VIII) 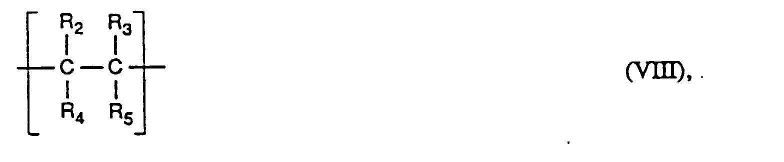 où les valeurs R₂ et R₃ sont chacune indépendantes de l'autre hydrogène, C₁-C₆ alkyle qui est, ou n'est pas, remplacé par de l'halogène, cyano ou nitro, ou sont du phényle ou du naphthyle, qui sont, ou ne sont pas, remplacés par de l'halogène, C₁-C₄ alkoxy, hydroxy, cyano ou nitro, et les valeurs R₄ et R₅ sont chacune indépendantes l'une de l'autre un atome d'hydrogène ou d'halogène, C₁-C₁₂ alkyle qui est, ou n'est pas, remplacé par des atomes d'halogène, cyano ou nitro, ou sont du phényle, naphthyle ou benzyle qui sont, ou ne sont pas, remplacés par de l'halogène, cyano ou nitro. C₁-C₄ alkyle ou C₁-C₄ alkoxy, ou sont un radical sélectionné dans un groupe consistant en -OR₆, -COOR₇ et COR₈ ou, avec un radical divalent des formules (IX)-(XI), sont 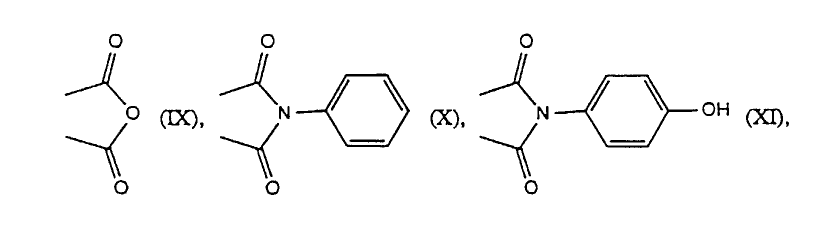 où les valeurs R₆ et R₇ sont chacune indépendantes de l'autre hydrogène, C₁-C₁₂ alkyle qui est, ou n'est pas, remplacé par de l'halogène, cyano ou nitro, ou sont du phényle ou du naphthyle, qui est, ou n'est pas, remplacé par de l'halogène, cyano, nitro, C₁-C₄ alkyle ou C₁-C₄ alkoxy, et R₈ a le même radical que R₆ ou est le radical 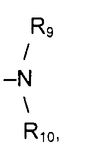 où les valeurs R₉ et R₁₀ ont chacune indépendamment l'une de l'autre la même signification que R₈.

5. Un polymère conformément à la revendication 4 contenant de 100 à 20 moles % d'une unité répétitive structurale de la formule (VII) et de 80 à 0 moles % d'une unité répétitive structurale de la formule (VIII).

6. Un polymère conformément à la revendication 4 contenant une unité répétitive structurale de la formule (VII), où R₁ est de l'hydrogène, n est 2 et les deux substituants OR sont dans la position 3,4- ou 3,5- par rapport au groupe vinyle.

7. Un polymère conformément à la revendication 4, où R dans la formule (VII) est tert-butoxycarbonyle, 2-tétrahydrofuranyle ou 2-tétrahydropyranyle.

8. Un processus pour la préparation d'un polymère conformément à la revendication 4, qui comprend la sujétion d'un composé de la formule (I) conformément à la revendication 1 ou un mélange d'un composé de la formule (I) et de composés de la formule (XII) 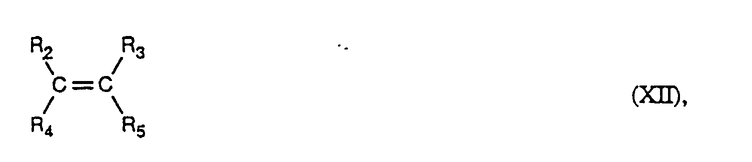 présents à raison d'un montant comportant un maximum de 95 moles %, où les valeurs R₂, R₃, R₄ et R₅ sont telles que définies dans la revendication 4 pour la polymérisation radicale.

9. Une composition sensible aux rayonnements comprenant (a) un polymère tel que revendiqué à la revendication 4 et (b) une substance qui produit un acide lorsqu'elle est exposée à des rayonnements actiniques.

10. Une composition conformément à la revendication 9 dans laquelle le composant (b) est un sel iodonium, sulfonium ou sulfoxonium.

11. Une composition conformément à la revendication 9 dans laquelle le composant (b) est sélectionné dans le groupe consistant en triphénylsulfoniumhexafluoroarsenate, triphénylsulfonium hexafluoroantimonate ou triphénylsulfonium trifluorosulfonate.

12. L'emploi d'une composition telle que revendiquée à la revendication 9 en tant que agent photorésistant pour la fabrication de circuits intégrés.
